# EUROPÄISCHE PATENTSCHRIFT

(11) **EP 0 770 372 B1**
(45) Veröffentlichungstag und Bekanntmachung des Hinweises auf die Patenterteilung: **01.03.2006**
(21) Anmeldenummer: 96117104.8
(22) Anmeldetag: 24.10.1996
(51) Int. Cl.: A61H 1/02, A61F 5/01, A61G 5/14

(54) **Orthopädisches Bewegungssystem**
Orthopedic motion system
Système de mouvement orthopédique

(30) Priorität: 25.10.1995 DE 19539680
(43) Veröffentlichungstag der Anmeldung: 02.05.1997
(73) Patentinhaber: Halsig, Peter, 88276 Berg (DE)
(72) Erfinder: Halsig, Peter, 88213 Ravensburg (DE)
(74) Vertreter: Otten, Herbert

(56) Entgegenhaltungen:
- EP-A- 0 109 572
- EP-A- 0 152 716
- EP-A- 0 502 792
- DE-U- 9 303 460
- FR-A- 2 329 251
- FR-A- 2 502 941
- FR-A- 2 665 074
- US-A- 5 176 706

## Beschreibung

Die Erfindung betrifft Bewegungssysteme in Form von Möbel , Trainingsgeräten oder Körperkorrektursystemen im Bewegungsraum Liegen - Sitzen - Stehen, sowie Bewegungen , die in diesen Stellungen durchgeführt werden können .
Ziel der Erfindung ist der möglichst anatomie - sowie physiologienahe Bewegungsablauf für das stauchungs - und verzerrungsfreie Bewegen für Rücken - Becken und Beine im Bewegungsraum Liegen - Sitzen - Stehen gsantriebe oder körperlicher.

Wie in den europäischen Patentschriften EP 0 311 993 sowie EP 0 591 668 beschrieben , bedarf es der ständigen Veränderung des Körpers durch Bewegung im Raum sowie der Bewegung von Teilen des Körpers zu - und gegeneinander und somit der Aktivierung seiner Regelorgane , um Anpassungen an die Umwelt oder interne Veränderung vornehmen zu können .
Das Dokument FR 2665074 stellt ein Schalensystem dar , das aus einem oder mehreren unterschiedlichen Anteilen bestehen kann , die den Körper eines Nutzers vom Kopf bis zu den Knien stützen . Im Besonderen kann die Schale zwischen Rücken und Sitz getrennt werden, sodaß eine unterschiedliche Winkeleinstellung der Rücken - zur Sitzfläche resultiert, die mittels verschiedener Stützsysteme gesichert werden kann. Die Verbindung beider Schalenanteile wird durch Scharniere hergestellt , welche die Schalenanteile in der Ebene der Schnittkante gegeneinander bewegen. Im Weiteren kann das System mit verstellbaren Rückenpositionierungen sowie einer Kopfstütze ausgerüstet sein . Ziel dieses Systems ist die unterschiedliche Positionierung eines Nutzers im Raum , sowie eine variable Einstellung des Rückens zum Sitz , was durch das Verändern des Winkels in der Schnittebene geschieht. Eine weitere Besonderheit ist die eingearbeitete , nichtgelenkig starre Oberschenkelführung innerhalb der Oberschenkelschale , hauptsächlich im Kniebereich , welche das gegenseitige Berühren der Oberschenkel , insbesondere der Kniegelenke verhindert. Da sich die Veränderung des Winkels zwischen Rücken - und Sitzfläche bei dem unter FR 2665074 beschriebenen Schalensystem an anderer Stelle befindet als das zu bewegende Hüftgelenk des Nutzers , nehmen die bewegten Anteile des Körpers eines Nutzers einen anderen Bewegungsweg als das Schalensystem , weshalb dieses System ein Positionierungssystem ist , da bei Winkeländerung zwischen Rücken - und Sitz vorhandene Zusatzeinrichtungen , wie eine Kopfstütze oder Rumpfstützsysteme - Pelotten - ihre ursprüngliche Position am Körper verändern und somit ihre zugedachte Stütz - oder Korrekturfunktion nur durch Nachjustierungen behalten können .

In dem neu vorgestellten Bewegungssystem werden gelenkgebundene oder gelenkersetzende Unterteilungen der Sitzeinheit und Rückeneinheit vorgenommen mit dem Ziel der fort - währenden Führung und Lenkung eines Nutzers während eines Bewegungsvorganges . Im Bereich einer Sitzeinheit werden die Unterteilungen entgegen den bisherigen Sitzein richtungen nicht zwischen Sitzteil und Rücken vorgenommen , sondern gemäß den anatomischen Verhältnissen besteht eine vollständig selbstständige Beckeneinheit , welche von der vollständigen Sitzbeinauflage bis zu den Beckenkämmen reicht . Die Beckeneinheit kann in ein bis drei Ebenen und unter Beachtung mehrerer Prinzipien bewegt werden , mit dem Ziel , den Rumpf , speziell die Wirbelsäule und die Hüftgelenke aktiv oder passiv bei entsprechender Anwendung zu einer angepassten Gegenbewegung gegen die Becken - bewegung zu bringen . Diese Beckeneinheit ist auf unterschiedliche Art und Weise durch gelenkersetzende oder gelenkgebundene anatomisch - physiologisch horizontal - und vertikalachsige Drehpunkte mit einer Oberschenkelplatte oder zwei Oberschenkelauflagen gelenkig verbunden. Die Beckeneinheit kann mit einem starren oder beweglichen Rücken verbunden sein .

Die Sitz - Rückenteile können zusätzlich im Sinne eines Modulbausatzes so gestaltet werden , daß den individuellen Bedürfnissen entsprechend nicht nur die Bewegungsebenen zusammenstellbar sind , sondern daß den individuellen Größenverhältnissen entsprechend die den Gesamtsitz tragende Beckeneinheit in der Breite sowie der Beckentiefe adaptiert werden kann , sowie eine adaptierbare Rückeneinheit und Beinbewegungsmodule drehpunktgerecht aufzunehmen imstande ist .

Die eventuell erforderlichen Antriebe können pneumatisch , hydraulisch, bowdengezogen , spindelgetrieben oder anderweitig motorischer Art sein .

### Erläuterung :

Sämtliche in der Erläuterung dargestellten Anwendungen sind Beispiele .
Die dargestellten Funktionsträger können in unterschiedlichen Bewegungsprojekten zur Anwendung kommen und sind nicht projektspezifisch .

In der Figurengruppe 1 wird entsprechend dem Anspruch 1 der Kernsitz , seine Bewegungs - sowie Anbindungsprinzipien dargestellt.

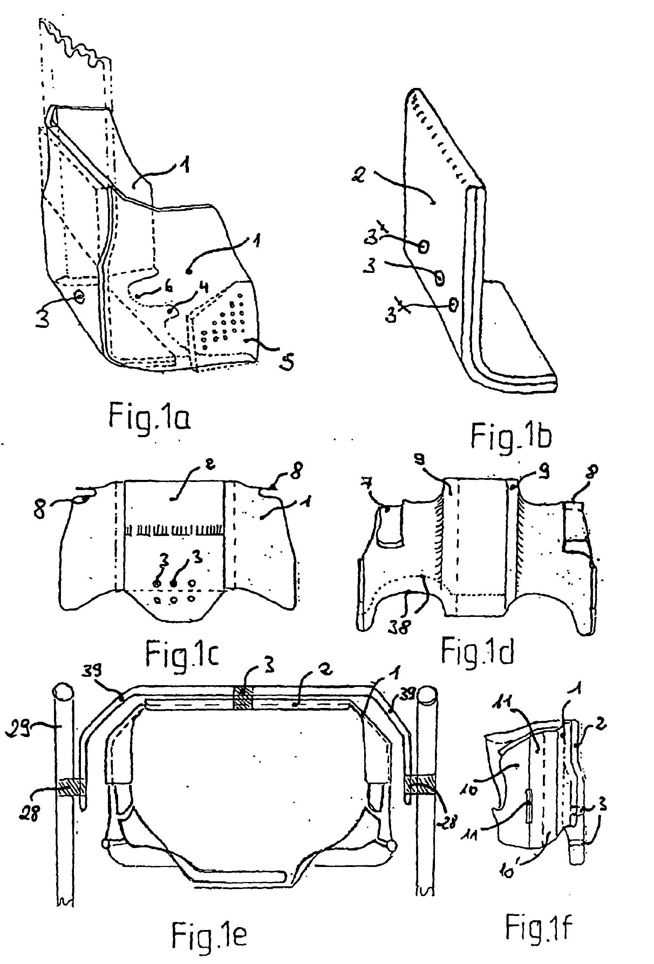

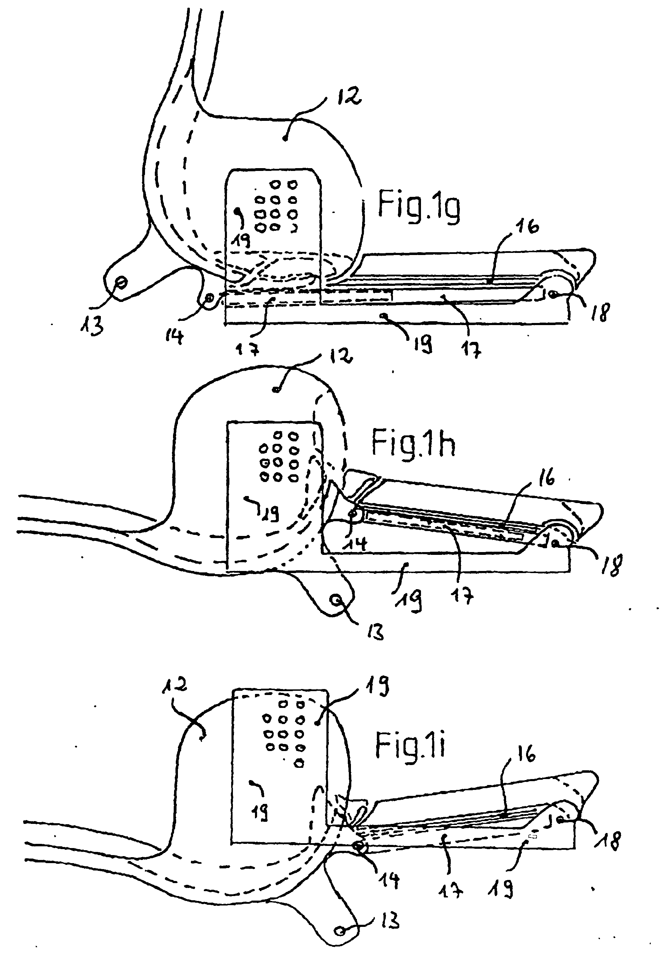

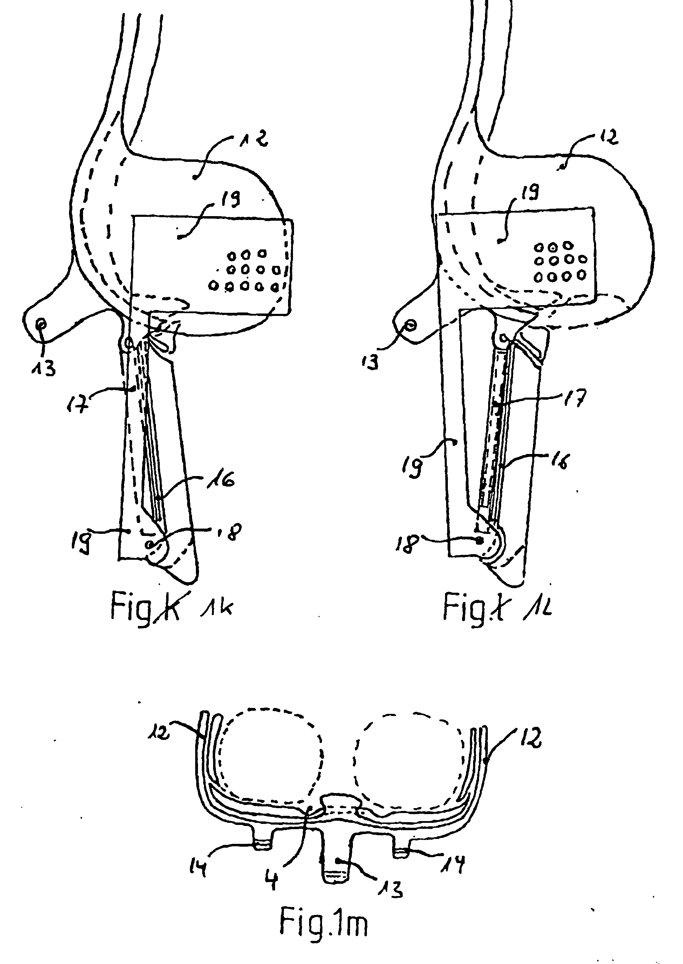

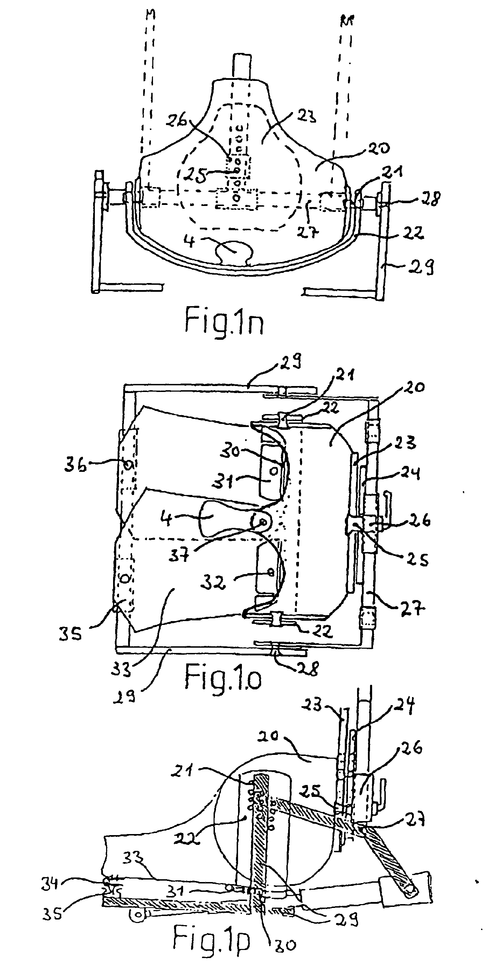

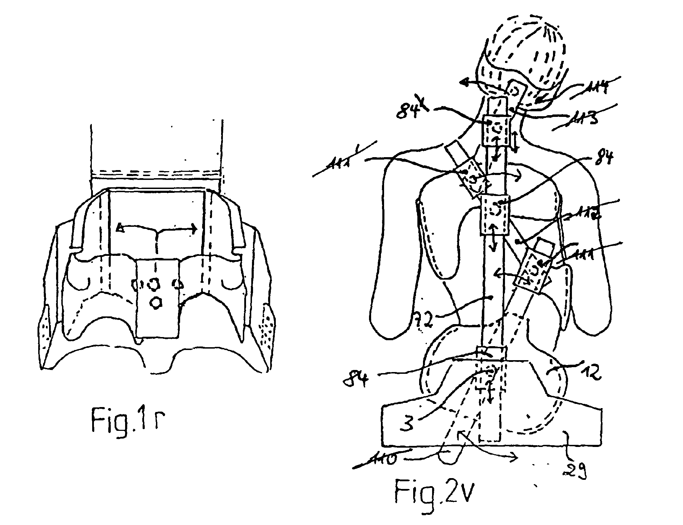

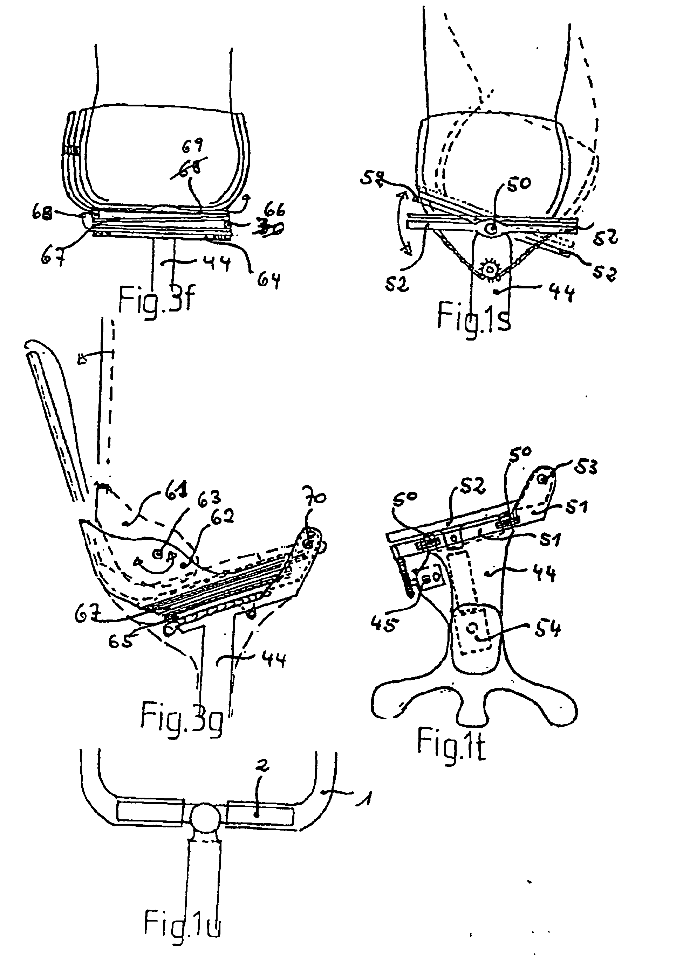

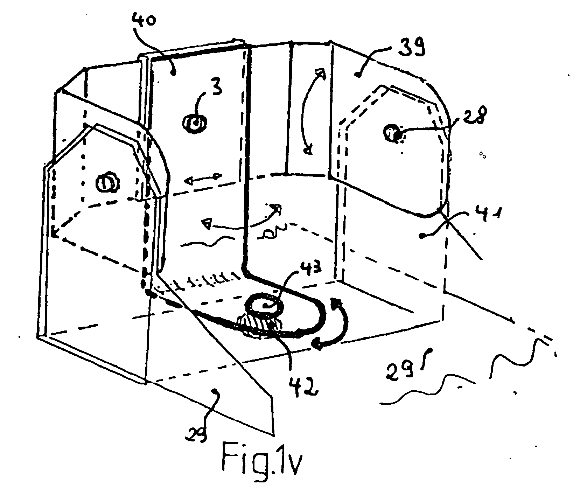

Fig 1a stellt eine modulare Beckeneinheit dar , mit deren Drehpunkteinrichtungen :
- in der Sagitalachse des oder der technischen Beckenseitbewegungsdrehpunkten ( 3 ).
- in der Horizontalachse des oder der technischen Hüftdrehpunkten

In dieser Beckeneinheit mit deren rechter und linker Beckenschale ( 1 ) sind in den seitlich , unteren Schalenanteilen die Gelenkflächen ( 5 ) für die Regelung des technischen . Hüftdrehpunktes integriert . Die Schnittkante der Beckeneinheit im Sitzbereich ist für die Liege und / oder Stehposition durch den Oberschenkelausschnitt ( 6 ) sowie die Sitzbein - Tuberauflage ( 4 ) gekennzeichnet .
Die Figurengruppe 1g-1m zeigt eine der drei Anbindungsprinzipien der Beckeneinheit an den Oberschenkelträger in Verbindung mit zwei Bewegungsprinzipien der Horizontal - achsenbewegung , dem Beckenhub , sowie der Beckenrückstellung :
1. die Teleskopverkürzung der Oberschenkelauflage mit unterschiedlichen technischen Hüftdrehpunkten :
   Fig. 1h : die Beckeneinheit ( 12 ) - Liegeposition -
   Fig. 1l : die Beckeneinheit ( 12 ) - Stehposition - ist über die technische Hüftdrehpunktfläche im Drehpunkt 10: 10, dem Beckenhub sowie
   Fig. 1i : die Beckeneinheit ( 12 ) - Liegeposition -
   Fig 1k : die Beckeneinheit ( 12 ) - Stehposition - ist über die technische Hüftdrehpunktfläche im Drehpunkt 10 : 3 , der Beckenrückstellung
      mit dem Sitzgrundgestell ( 19 ) und dessen Drehpunktfläche gelenkig verbunden. Zur Aufnahme etwaiger Beckenbewegungsantriebe kann die Beckeneinheit z.B.:mit einem Antriebsflansch ( 13 ) für einen Spindelantrieb und zwei Aufhängungen ( 14 ) für eine linke und rechte Teleskopführung ( 17 ) ausgestattet sein . Diese Teleskopfiihrungen sind eine von dreien Anbindungsprinzipien des Oberschenkelequipments . Sie dienen zur Aufnahme einer oder zweier Oberschenkelplatten ( 16 ) und sind mit dem Sitzgrundgestell ( 19 ) im technischen Kniedrehpunkt ( 18 ) gelenkig verbunden . Die physiologischen Auswirkungen der beiden Bewegungsprinzipien lassen sich unschwer erkennen :
      Der Beckenhub der oben genannten Einstellung geht sowohl im Liegen ( Fig.1h ) als auch im Stehen (Fig. 1l) mit einer extremen Hüftstreckung einher, wohingegen die Beckenrückstellung ( Fig. 1i im Liegen , sowie Fig. 1k im Stehen ) in beiden Ausgangspositionen eine entspannte Hüftgelenk - und Hüftmuskelhaltung erlauben.
      Die Fig. 1m zeigt einen Querschnitt durch die Beckeneinheit ( 12 ) mit Tuberauflage ( 4 ) , Antriebsflansch ( 13 ) und den beiden Teleskopführungsaufhängungen ( 14 )

   Die Figurengruppe 1n - 1p zeigt einen Kernsitz in einer Bewegungskombination in der Hafthorizontal-und Beckensagitalachse mit dem Bewegungsprinzip der Beckenseitschwenkung , der Bewegung in der Sagitalachse , dem Liegen oder Stehen , dem Oberschenkelanbindungsprinzip des Unterzuges , sowie einer geteilten , beidseitigen Oberschenkelführung in die Hüftabduktion für Bewegungen im Bewegungsraum Stehen - Sitzen - Liegen .
2. Dieser Sitz ist mit dem Oberschenkelanbindungsprinzip des Unterzuges versehen und besteht aus der Beckeneinheit ( 20 ) mit dessen Oberschenkelausschnitten und dem integrierten Drehpunkt ( 37 ) zur Aufnahme des schwenkbaren Sitzbein - Tubersattels (4) sowie der Beckenrotationsachse. Die Beckeneinheit ist mit der Drehpunktplatte ( 23 ) und ein oder mehreren Drehpunkten <25> für eine Beckenseitschwenkbewegung, integrierter Aufnahmefläche <21> zur gelenkigen Anbindung des Unterzuges ( 22 ) an den technischen horizontalachsigen Drehpunkt, dem Hüftdrehpunkt des Sitzes ausgestattet. Die mittels Scharnier ( 30 ) mit dem Unterzug ( 22 ) im Oberschenkelausschnitt verbundenen Ober - schenkelklappen ( 31 ) dienen zur gelenkigen Anbindung <32> der Oberschenkel - platten oder - schalen ( 33 ) an den Unterzug ( 22 ). Die Oberschenkelschalen sind mit dem Sitzgrundgestell ( 29 ) durch die Achse ( 36 ) über ein Scharnier ( 34 ), welches an einer horizontalachsigen Teleskopführung ( 35 ) befestigt ist , verbunden , was in jeder Becken - oder Oberschenkelraumlage eine Abspreizung - Abduktion - der Oberschenkel erlaubt .
   Die Anbindung der Beckeneinheit ( 20 ) über die Drehpunktplatte ( 23 ) und den Drehpunkt ( 25 ) auf das Sitzgrundgestell ( 29 ) erfolgt über die Drehpunkt - aufnahmefläche ( 24 ) des Höhenanpassadapters ( 26 ) am Becken - Rückenauf - nahmerahmen ( 27 ), der im Hüftdrehpunkt ( 28 ) mit dem Sitzgrundgestell ( 29 ) verbunden ist .
   Der Höhenanpassadapter ( 26 ) ist für die Grundanpassung der Beckeneinheit ( 20 ) bei verändertem Seitschwenkdrehpunkt ( 25 ) verantwortlich .
   Fig . 2 v zeigt eine bewegungsintegrierte Kernsitzeinheit, die nach dem Prinzip der Beckenseitschwenkung arbeitet , bei welcher über eine in das Sitzgrundgestell ( 29 ) integrierte Zentralsäule ( 72 ) eine an einem Schiebe - oder Gleitadapter ( 84 ) , der auch feststellbar sein soll , über das Gelenk ( 3 ) verbundene Steuerstange ( 110 ), die eine Becheneinheit ( 12 ) , die über das Gelenk ( 3 ) beweglich oder alternativ mit der Steuerstange fest verbunden , im Prinzip Seitschwenkung bewegt.

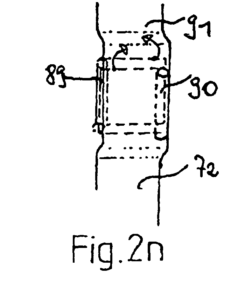

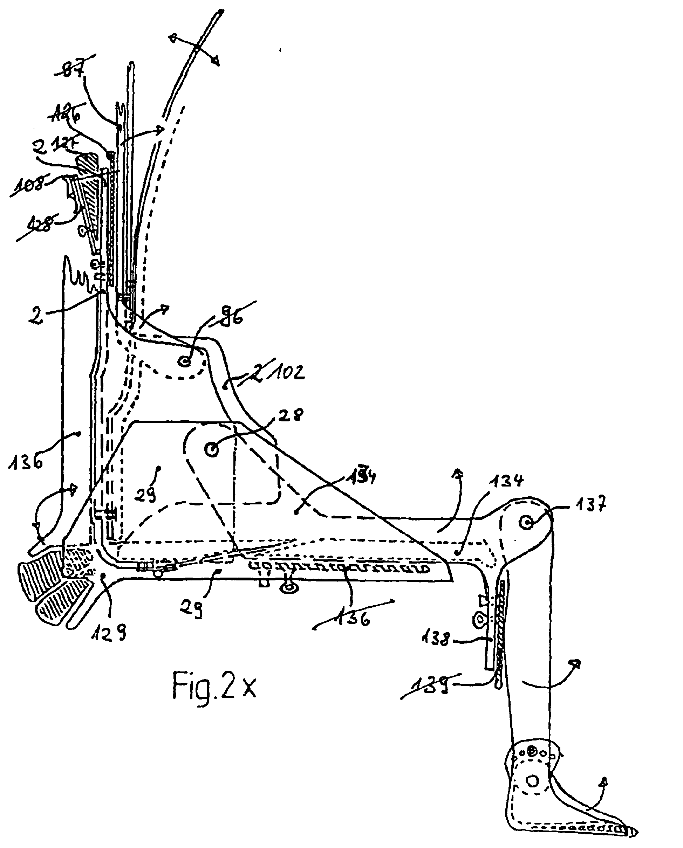

3. Fig 2 x : Anbindungsprinzip der direkten Anbindung der Oberschenkelauflage an den technisch anatomisch - physiologischen Hüftdrehpunkt , was die Kontinuität der Oberschenkelauflage sowie der Knieebene über den Bewegungsablauf wahrt: Kernsitz , bestehend aus Beckeneinheit (102) und mit dieser im Drehpunkt ( 28 ) gelenkig verbundenen Oberschenkelplatte ( 134 ) , mit Antriebsplatte ( 138 ) für die Unterschenkelschale , die mit der Oberschenkelplatte ( 134 ) über den Drehpunkt ( 137 ) gelenkig verbunden ist.
   Der Kernsitz ist mit dem Sitzgrundgestell ( 29 ) im Drehpunkt ( 28 ) gelenkig verbunden . Der notwendige Kemsitzantrieb im Drehpunkt (28 ) erfolgt in diesem Beispiel über eine in das Sitzgrundgestell ( 29 ) integrierte , lufttaschenbestückte Flächenstütze ( 129 ) , dessen Gegenstück aus der Flächenstütze einer im Kernsitz integrierten Mittelsäule ( 136 ) besteht. Diese Antriebsanordnung ermöglicht das Ausnutzen von Bewegungsrestfunktionen und den Bewegungsfolgeantrieb in jede Nutzerstellung .

In der Figurengruppe 1s ( Ansicht von hinten ) und 1t ist das Bewegungsprinzip einer Beckenseitsenkbewegung beispielsweise über eine Kettenansteuerung dargestellt.
In einer Zentralsäule ( 44 ) oder einer zentralen Doppelscheibe mit mittelstehendem Spindelmotor ( 54 ) welcher zur Sitzneigungsverstellung in der Horizontalachse die Sitzaufnahmeplatte ( 52 ) über den Kniedrehpunkt ( 53 ) durch eine Aussparsparung in der Grundplatte ( 51 ) antreibt , welche die Seitsenkbewegung des Beckens über Drehpunkt ( 50 ) - Fig.1s als einlagiger Drehpunkt , Fig. 1t als 2 fach gelagerter Drehpunkt - aufnimmt , ist zu dessen Antrieb beispielsweise ein zahnkranzbestückter Motor ( 45 ) in die Mittelsäule oder das Doppelplattensystem integriert , welcher über eine an der Grundplatte ( 51 ) beidseits befestigte Kette die Seitsenkbewegung des Beckens führt .
Fig .1 u : zeigt eine andere Form der breitenanpassbaren Kassetierung , bei welcher der Kassettenkern ( 2 ) innen liegt und die Seitenteile ( 1 ) außen gleiten.
Fig 3 f und 3 g zeigen einen Bewegungsstuhl mit Faltblattentechnik , dessen Bewegungsprinzip dem des Beckenhubes oder Beckenrollens entspricht .
Fig. 3 f Ansicht von hinten stellt die dritte der Beckenbewegungsprinzipien inder Sagitalachse dar : den asymmetrischen Beckenhub : Bei dieser Technik liegen die technischen Bewegungsdrehpunkte jeweils im seitlichen Gesäßbereich .
Fig . 3f ( Rückansicht ) und Fig 3 g . zeigen einen Bewegungsstuhl nach dem Bewegungsprinzip des asymmetrischen Beckenhubes oder Beckenrollens in der Seitbewegung in der Sagitalebene :
   An einer Zentralsäule (44) mit der Grundplatte ( 64 ) ist über den Drehpunkt ( 70 ) die lufttaschenbestückte Sitzflächen neigungsplatte ( 65 ) verbunden, an der über ein längsverlaufendes Scharnier ( 66 ) die Seithebeplatte ( 67 ) gelenkig angebunden ist . Mit dieser ist über das gegenüberliegende Scharnier ( 68 ) die anderseitige Seithebeplatte ( 69 ) und / oder ein Sitzträger ( 62 ) verbunden , mit dessen technischem Hüftdrehpunkt ( 63 ) eine Beckeneinheit ( 61 ) mit oder ohne Rückenplatte gelenkig verbunden ist .
   Ein solcher Stuhl kann zu therapeutischen wie zu Trainingzwecken das Becken in zwei Ebenen zwangsbewegen , wobei der Nutzer über die Becheneinheit ( 61 ) reaktiv den Rumpf im Gleichgewicht halten oder anpassen lernt.

Wenn nun die drei Beckenseitbewegungsprinzipien gegenübergestellt werden:
a.) die Beckensenkung ( Fig . 1s ) mittig tiefer Drehpunkt
b.) der asymmetrische Beckenhub ( Fig . 3 f ) seitlich tiefer Drehpunkt
c.) die Seitschwenkung (Fig . 1n ― 1p) mittig - mittlerer bis hoher Drehpunkt ( oberes Kreuzbein bis Lendenwirbelsäule )
   so lässt sich erkennen, dass die anatomisch - physiologischen Auswirkungen auf den Rumpf, speziell die Wirbelsäule gänzlich unterschiedlich sind .
Fig. 1c - 1f sowie Fig . 1r und Fig . 1v stellt ein breiten - und tiefenanpassbares modulares Beckenbewegungssystem dar , das in konventionelle Sitzsysteme vornehmlich bei Behinderten integriert werden kann, mit der sagitalachsigen Primärfunktion Beckenseitbewegung und den über Adaptermodule möglichen Optionen der horizontalachsigen Beckenaufrichtung und - kippung sowie der vertikalachsigen Beckenrotation gegenüber der Rumpf - und Oberschenkelachse : dem kassettenkern
   Fig . 1c ( Ansicht von hinten ) zeigt das Modul mit ( 2 ) , den in diese einsteckbaren Bechenschalen oder Seitenteilen ( 1 ), den in die Kassette integrierten möglichen Drehpunkten ( 3 und ff.), sowie der für den Anwendungsbereich wichtigen Darmbeinfixation ( 8 )
   Fig 1d ( Ansicht von vorne ) vorderes Modulteil mit der Darmbeinfixation , einmal ohne Rippenlager ( 7 ) oder mit Rippenlager ( 8 ) den Bauchplatten ( 9 ), welche z.B.:in der Stehständerversion in Verbindung mit der Kassette die Breitenanpassung von vorne und somit den Einstieg von hinten ermöglichen . Die Oberschenkel - aussparungen ( 38 ) für das Sitzen können per Schnittkante den individuellen Verhältnissen angepasst werden.
   Fig. 1f Modul von der Seite mit Kassette ( 2 ) den Drehpunkten ( 3 ) den Seitenteilen ( 1 ), welche bis zur Gelenkschnittfläche des Vorderseitenteiles ( 10 ) , welches wiederum mit der Adapterfläche (10') über das Scharnier (11) verbunden, bis zur Kassettenebene der Seitenteile reicht .
   Fig. 1e Ansicht von oben und mit Adapter ( 39 ) im Drehpunkt (3) verbunden. Der Adapter ist über die seitlichen Drehpunkte ( 28 ) mit dem Sitzgrundgestell ( 29 ) verbunden , was eine Beckenseit - und Kippbewegung : Liegen - Stehen ermöglicht .
   Fig .1v zeigt einen weiteren Adapterkomplex , mit einem Rotationsadapter ( 41 ), der über den Drehpunkt ( 42 ) mit dem Grundgestell ( 29 ) verbunden , den Kippadapter ( 39 ) im Drehpunkt ( 28 ) aufnimmt , welcher über den Drehpunkt ( 3 ) den Seitschwenkadapter ( 40 ), der alternativ zum Drehpunkt ( 42 ) über den eigenen Drehpunkt ( 43 ) den Kernsitz aufnimmt , was eine dreidimensionale Bewegung oder Positionierung des Beckens eines Probanden ermöglicht.
   Fig.2n stellt ein Rollmodul , bestehend aus zwei gegenseitig erbeitenden , senkrecht oder schräg stehenden Scharnieren dar , bestehend aus der Mittelsäule ( 72 ) mit integriertem Scharnier zur Linksdrehung ( 89 ) und dem gegenläufigen Scharnier ( 90 ) zur Rechtsdrehung , an das die obere Mittelsäule ( 91 ) befestigt ist
   Fig . 1r zeigt das Beckenbewegungssystem ( Fig.1c - f ) in einem Kernsitz ( Fig. 1a - b ) integriert , wobei der Kernsitz die Beckenbewegung in der Horizontalachse und das modulare Beckenbewegungssystem die Bewegung der Sagitalachse übernimmt.

## Patentansprüche

1. Orthopedisches Bewegungssystem mit einem für die Aufnahme eines Probanden bestimmten Sitz, der aus einer vom Bekkenkamm bis zur vollständigen Sitzbeinauflage reichenden Beckeneinheit (12, 20) besteht, und mit wenigstens einer an die Beckeneinheit angelenkten Oberschenkel-Auflage (16, 33),
**dadurch gekennzeichnet, daß** das Bewegungssystem eche Vorrichtung zum Längenansgleich zwischen Beckeneinheit (12,20) und Oberschenkel-Auflage (16, 33) aufweist, wobei die Vorrichtung zum Länger ausgleich entweder zwei an der Beckereinheit angeordnete Hüfldrehpunkfläches (5) mit mehreren Bohrungen zur individuell anpaßbaten Anleskung der Oberschenkelauflage (16,33) an der Beckereinheit oder einer Pendel unterzug (22) für die Oberschenkelauflage (16, 33) oder eine Teleskopweskrüszung (17) aufweist, so daß die Beckeneinheit (12, 20) ohne Scherung oder Stauchung im Bereich des Beckens, der Wirbelsäule und der Sitzbeine eines in der Beckeneinheit aufgenommenen Probanden gegen die Oberschenkel-Auflage (16, 33) bewegbar ist,

2. System nach Anspruch 1, **gekennzeichnet dadurch , dass** die Oberschenkelauflage ( 33 ) mit der Beckeneinheit (12,20) durch Gelenke ( 37 , 42 , 43 ) im Sinne einer Oberschenkel-abspreizung und Beugung und Streckung zweidimensional gelenkig verbunden ist.

3. System nach Anspruch 1 oder 2, **dadurch gekennzeichnet, dass** das Bewegungssystem als Modul aufgebaut ist, bestehend aus einer oder mehreren Kassetten ( 2 ), welche eine individuelle Breiten - und Tiefenanpassung im Bereich des Beckens , durch die Führung seitlicher ( 11 ), wie vorderer Module ( 10 ) , die zudem im Bereich der Beckenkämme ( 8 ) und im Darmbeinstachelbereich anatomisch angeformt sein können , ermöglichen, sowie die für die Beckenbewegung drehpunktgerechte Einbindung von Rücken - und Beinbewegungsmodulen erlauben.

## Claims

1. Orthopaedic motion system with a seat designed to hold a patient, which comprises a pelvic unit (12, 20) extending from the iliac crest to the complete ischium rest, and with at least one thigh rest (16, 33) hinged onto the pelvic unit,
**characterised in that** the motion system comprises a device for adjusting the length between the pelvic unit (12, 20) and the thigh rest (16, 33), whereby the device for adjusting the length comprises either two hip pivot sides (5) arranged on the pelvic unit with several holes for individually adjusting the hinging of the thigh rest (16, 33) onto the pelvic unit or a pendulum bearer (22) for the thigh rest (16, 33) or a telescopic shortening device (17), so that the pelvic unit (12, 20) can be moved against the thigh rest (16, 33) without causing shearing or compression in the region of the pelvis, vertebrae and ischium of a patient sitting in the pelvic unit.

2. System according to claim 1, **characterised in that** the thigh rest (33) is pivotably connected with the pelvic unit (12, 20) in two dimensions by means of hinges (37, 42, 43) for the purpose of spreading out the thighs, bending and stretching.

3. System according to claim 1 or 2, **characterised in that** the motion system is constructed as a module, comprising one or more units (2) which permit individual width and height adjustments in the region of the pelvis by guiding lateral (11) and front modules (10), which can also be shaped anatomically in the region of the iliac crests (8) and in the iliac spine area, and also permit the pivotally correct binding of the back and the leg movement modules for the movement of the pelvis.

## Revendications

1. Système de mouvement orthopédique avec un siège destiné à recevoir un sujet, le siège se composant d'une unité de bassin (12, 20) s'étendant depuis la crête du bassin jusqu'à l'appui complet de l'ischion, et d'au moins un support de cuisse (16, 33) articulé sur l'unité de bassin,
**caractérisé en ce que**
le système de mouvement présente un dispositif de compensation en longueur entre l'unité de bassin (12, 20) et le support de cuisse (16, 33), le dispositif de compensation en longueur présentant deux surfaces de point de rotation (5) avec plusieurs perçages disposés sur l'unité de bassin, pour une articulation adaptable individuellement du support de cuisse (16. 33) à l'unité de bassin, ou une traverse oscillante (22) pour le support de cuisse (16, 33) ou un raccourcissement télescopique (17), de sorte que l'unité de bassin (12, 20) soit mobile contre le support de cuisse (16, 33) sans cisaillement ou déformation sous pression dans la zone du bassin, de la colonne vertébrale et des ischions d'un sujet installé dans l'unité de bassin.

2. Système selon la revendication 1.
**caractérisé en ce que**
le support de cuisse (33) est relié de manière articulée bidimensionnelle à l'unité de bassin (12, 20) par des joints (37, 42, 43) selon un écartement de la cuisse, un pliage et une extension.

3. Système selon la revendication 1 ou 2,
**caractérisé en ce que**
le système de mouvement est monté en tant que module, se composant d'une ou de plusieurs cassettes (2) qui permettent une adaptation en largeur et en profondeur dans la zone du bassin, par le guidage des modules latéraux (11), comme des modules avant (10), qui peuvent en outre être moulés de façon anatomique dans la zone des crêtes du bassin (8) et dans la zone de l'épine iliaque et permettent l'insertion des modules de mouvement du dos et des jambes par rapport au point de rotation pour le mouvement du bassin.
